# EUROPEAN PATENT APPLICATION

(11) **EP 3 974 019 A1**
(43) Date of publication of application: **30.03.2022**
(21) Application number: 20461562.9
(22) Date of filing: 29.09.2020
(51) Int. Cl.: A61N 1/04

(54) **A SURFACE ELECTRODE**

(71) Applicant: Neuro Device Group S.A., 04-501 Warszawa (PL)
(72) Inventor: Giergielewicz, Mariusz, 04-501 WARSZAWA (PL); Prokopczyk, Pawel, 04-501 WARSZAWA (PL); Jankiewicz, Adam, 04-501 WARSZAWA (PL); Malej, Krzysztof, 04-501 WARSZAWA (PL)
(74) Representative: Kancelaria Eupatent.pl Sp. z.o.o

(57) **Abstract**

A surface electrode for application on a skin, the surface electrode comprising electrode units (10) connectable to a voltage and/or current supply. Each electrode unit (10) comprising a substrate (12) having a front side (12a) and a back side (12b), and component electrodes (11) disposed on the front side (12a) of the substrate (12) and spaced apart from each other such that a free space volume (111) is formed between the component electrodes (11), each component electrode (11) having a proximal end (11b) connected with the substrate (12), a distal end (11a) configured to be applied on the skin, and a medium portion (11c) between the proximal end (11b) and the distal end (11a).

## Description

### TECHNICAL FIELD

The present invention relates to a surface electrode for non-invasive electrostimulation including electrostimulation of desired body parts, including, head, arms, legs, bag, organs, tissues and/or tissues cells, e.g. brain, nerve system, nerve cells, muscles, skin, epidermis etc.

### BACKGROUND

A surface electrode is a device connectable to a stimulation unit which constitutes an electricity source. During the electrostimulation, the surface electrode stays on a skin external surface, playing an important role in interfacing the skin tissue with the stimulation unit to which it is connected. The surface electrode may be attached to the skin, e.g., the electrode for use on head, may be attached using cap or headbands. When current, typically consisting of a series of electrical waveforms, is applied to the surface electrode, placed on the skin substantially overlaying the nerve structures, an electric field is generated between two electrodes (anode and cathode) and ions which creates a current in the desired place of the body part or tissue.

In other words, the surface electrode constitutes a terminal through which electrical current passes into the underlying tissue. At the electrode-tissue interface a conversion occurs between the current of electrons (driven from the stimulation unit through the electrode) and the current of ions in the tissue.

Engineering of the surface electrodes aims at proper user's cutaneous sensation during the electrostimulation, including the absence of skin burns and minimized skin irritation, along with a proper electro-stimulation of the target element(s) of the body as the design of the surface electrode shapes the current flow through the target element(s) of the body.

Depending on the given treatment requirements, the surface electrodes are optimized for a low impedance. An impedance that is too high leads to elevated voltage resulting in increased irritation of the skin followed by skin burns.

The surface electrodes can be of various shapes, wherein typical shapes include snap, pin, pellet, disk, sheet, or mesh, with the electrode body made of metal or conductive rubber. The shape of the electrode can be adjusted to application. Generally the smaller the electrode size, the higher the current density. Thus, the electrode size is a factor limiting the maximum amplitude of stimulation current/voltage.

An important aspect of the electrostimulation with the surface electrode is the conductive medium used at the electrode-tissue interface. The preferred conductive medium is an electrolyte, typically in a liquid, semi-liquid or solid form, such as saline, conductive paste, or conductive gel. The conductive medium forms a conductive layer between the user's skin and the electrode body providing therein a conversion of electrons from the electrode into appropriate ions. The conductive layer should be present during the whole period of the electrostimulation to avoid skin irritation and burns. However, this is difficult to achieve, as the conductive medium can evaporate during the duration of the electrostimulation, causing discontinuities in the conductive layer. An assembly of the surface electrode typically comprises holding means for holding the conductive medium in a form of a continuous conductive layer. The holding means typically include sponge materials soaked with the conductive medium. Nonetheless, the latter require periodic feeding with a fresh dose of saline dose, due to the evaporation of saline, which imposes various technical difficulties with feeding the sponges with saline.

Further, there exist self-adhesive electrodes, comprising the conductive medium, a gel or a hydrogel formed into the conductive layer to cover the electrode surface. This conductive layer is rigid and may be attached directly, or with the aid of an adhesive, to the electrode surface.

Yet further, there exist dry electrodes that do not use external conductive medium. Instead, the dry electrodes use sweat from the user's skin. These electrodes are optimized for work with high impedance. However, high-impedance solutions may work reasonably well in biosignal recording, while in case of electrostimulation the use of current flow between high-impedance electrodes leads to elevated voltage resulting in increased irritation of the skin followed by skin burns. Impedance of dry electrodes is especially high shortly after positioning of electrodes on the skin, thus limiting possibility to even start electrostimulation. After some time impedance gets better due to sweating, wherein sweat may act as conductive medium.

Good quality of the surface electrode operation mode is further accomplished by providing a substantially large contact surface between the electrode, the conductive medium and the skin. However, this is difficult to achieve due to the electrode shape, which typically does not correspond with uneven body shape, and may be even further diminished at hairy skin regions, such as scalp regions, e.g. during brain electrostimulation, due to the presence of hair which significantly reduce the above-mentioned contact surface.

The above aspects of surface electrode operation, i.e. the required large contact surface (electrode / conductive medium / skin) along with a proper application of the conductive medium onto the skin and long-lasting maintenance of the formed conductive layer, most preferably during the whole electrostimulation, are important factors which, if properly combined, can ensure good operational mode of the surface electrode, including minimized skin irritations together with selected impedance regime in the required application time.

Among the known architectures of the surface electrodes, there are known transcranial stimulation electrodes for scalp application and brain electrostimulation, especially neuromodulation of patients with brain injuries as well as electrostimulation of various psychiatric conditions like major depressive disorder. During the electrostimulation, the electrode stays in electrical contact with the stimulation unit, i.e. a current or voltage generator that generates stimulation current or voltage substantially transdermally delivered via the surface electrode, and further via the layer of conductive medium to the tissue. Since the conductive medium requires even distribution over the scalp hair, this transcranial electrode can be either equipped with a saline-soaked sponge, or it can be provided with an electroconductive paste to form the conductive layer of conductive medium. The proper formation of the conductive layer of conductive medium enables the electrode to achieve a low impedance operational mode. Nonetheless, the process for preparing the desired conductive layer of conductive medium is complex, time-consuming, as well as it requires the engagement of highly-qualified and experienced medical personnel. Therefore, the preparation and installation of the electrode can be carried out only at points of care, e.g. hospitals.

Proper positioning of the surface electrode on hairy skin regions, especially in the presence of dense and/or long hair is very difficult. Hair may form an additional undesired layer between skin and electrode, and further affects the formation and maintenance of the continuous conductive layer of conductive medium, during the electrostimulation. Typically, flat electrodes are difficult to apply in this condition. Alternatively there were proposed new designs of electrodes engineered as electrode units of the plurality of components or component electrodes usually of pin-like or spike-like shapes, attached to a common substrate forming a brush- or comb-like design. This design facilitates application, as the plurality of component electrodes can be positioned between hairs.

Nonetheless, the brush-like or comb-like design of the surface electrode encounters various problems associated mainly with the application of the conductive medium into the area of the skin-electrode interface, and further maintenance of the conductive layer during the whole electrostimulation to provide proper transfer of the electrical charges and to avoid skin irritation and burns. Furthermore, due to limited contact area of pins or spikes, a use of component electrodes can lead to undesirably increased impedance and excessive current density resulting in low simulation comfort and/or skin injuries. This drawback may be further amplified by uneven contact of the electrodes with the skin, as the units of component electrodes attached to the common substrate can be rigid, unfitting the body shape.

From the patent literature, there are known various designs of the surface electrodes for electrostimulation.

A European patent publication EP3142744 describes a transcranial stimulation electrode that consists of a super-porous hydrophilic material having a first relaxed state and a second expanded state. In the latter state, the super-porous hydrophilic material is breakable to at least partly enclose at least one hair. The current is driven from the electrode surface, through the hydrophilic material, to the hairy skin. The super-porous hydrophilic material of the electrode comprises polymerized acrylamide, with additives such as: polymerized acrylic acid, polymerized sulfopropylacrylate potassium salt, polymerized methylene bisacrylamide.

Taking into account the above drawbacks, the present invention aims to develop a surface electrode of an improved contact area: electrode / conductive layer of conductive medium / skin, as well as facilitated formation and maintenance of the conductive layer of a conductive medium during the whole electrostimulation period, either at the beginning of the treatment, as well as if prolonged. Furthermore, the present invention aims to provide the surface electrode of simplified handling enabling the user to utilize the surface electrode at home conditions, e.g. only under medical supervision or purely on medical instruction, depending on the individual considerations.

### SUMMARY

There is disclosed a surface electrode for application on a skin, the surface electrode comprising electrode units 10 connectable to a voltage and/or current supply. Each electrode unit 10 comprising a substrate 12 having a front side 12a and a back side 12b, and component electrodes 11 disposed on the front side 12a of the substrate 12 and spaced apart from each other such that a free space volume 111 is formed between the component electrodes 11, each component electrode 11 having a proximal end 11b connected with the substrate 12, a distal end 11a configured to be applied on the skin, and a medium portion 11c between the proximal end 11b and the distal end 11a.

Preferably, the surface electrode further comprises a system 2 for supplying a conductive medium to the free space volume 111, the system comprising a manifold 20 arranged on the back side 12b of the substrate 12, the manifold 20 comprising ducts 21 ended with outlet tubes 22 arranged in the substrate 12 from the back side 12b to the front side 12a for supplying the conductive medium to the component electrodes 11.

Preferably, the electrode units 10 are arranged on a deformable layer 30 that is disposed between the manifold 20 and the substrate 12 facing the back side 12b of the substrate.

Preferably, the deformable layer 30 has openings for disposing the outlet tubes 22 from one side of the deformable layer 30 to the other side.

Preferably, the manifold 20 is connected with the substrate 12 by fastening means 23, 121.

Preferably, the surface electrode comprises at least two electrode units 10.

Preferably, one electrode unit 10 comprises at least four component electrodes 11.

Preferably, the component electrodes 11 comprise the distal ends 11a of a distal end thickness (T) having a shape selected from the group consisting of sphere, hemisphere, roll, cylinder, stick-like, pin-like, spike-like, and pyramidal shape, the medium portions 11c of a medium portion thickness (t), and the proximal ends 11b having a shape selected from the group consisting of roll, cylinder, stick-like, pin-like, spike-like, and pyramidal shape, wherein, within one electrode component 11 the distal end thickness (T) is greater than the medium portion thickness (t).

Preferably, the component electrodes 11 are made of stainless steel or chloride plated with gold or with silver.

Preferably, the system for supplying a conductive medium is connected with a reservoir 24 for conductive medium.

Preferably, the system for supplying a conductive medium comprises a pump for feeding the conductive medium from the reservoir 24 to the outlet tubes 22.

Preferably, the deformable layer 30 is made of at least one material selected from the group consisting of cotton, polyamide, polyester and leather.

Preferably, the component electrodes 11 are an integral part of the substrate 12.

Preferably, the component electrodes 11 are attached to the substrate 12.

### BRIEF DESCRIPTION OF THE DRAWINGS

These and other objects of the invention presented herein are accomplished by providing a surface electrode as described herein. Further details of the electrode, its technical features and various advantages will become more apparent from the following detailed description of the preferred embodiments shown in a drawing, in which:
Figs. 1A - 1C present an electrode unit of a surface electrode according to the present invention;
Figs 1D - 1G presents a substrate of the electrode unit with component electrodes arranged therein;
Fig. 2 presents a system for supplying a conductive medium of the electrode unit of the surface electrode according to the present invention;
Fig. 3 presents an embodiment of the surface electrode according to the present invention;
Fig. 4 schematically presents a working principle of the surface electrode according to the present invention;
Fig. 5 presents exemplary fastening means of the electrode unit of the surface electrode according to the present invention, as well as external conductive medium reservoir.

### DETAILED DESCRIPTION

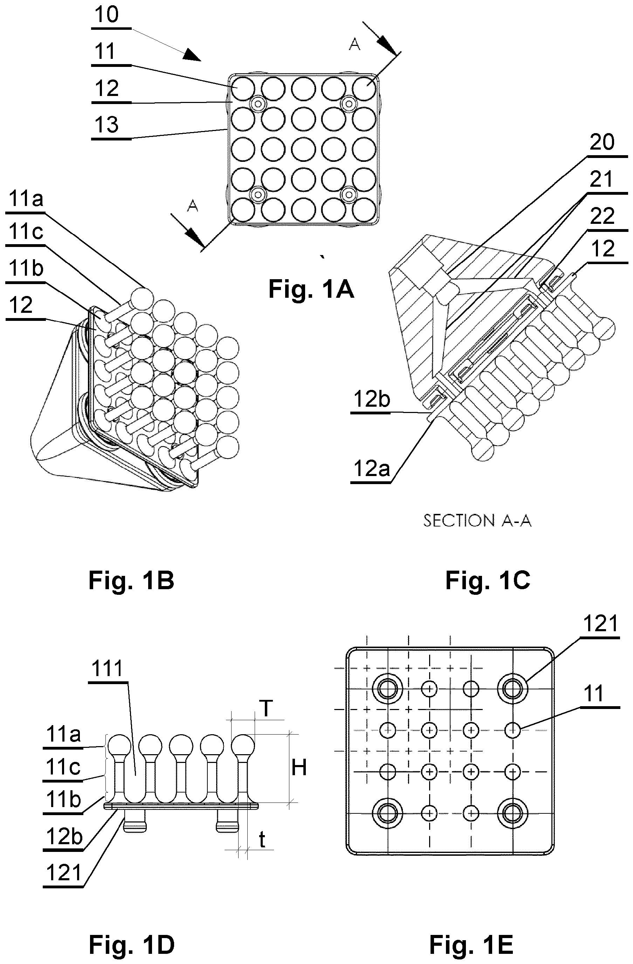

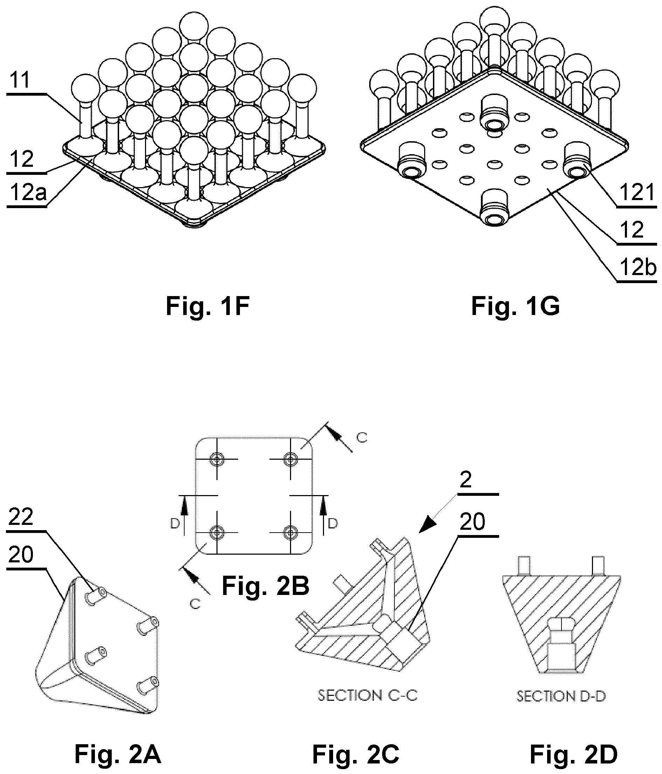

The surface electrode comprises at least one electrode unit 10, and more preferably a plurality electrode units 10. Each of the electrode units 10 comprises a plurality of component electrodes 11, preferably each of the same length, and disposed on a common substrate 12, as shown in Fig 1. The substrate 12 comprises a front side 12a on which the component electrodes 11 are arranged 11, and a back side 12b.

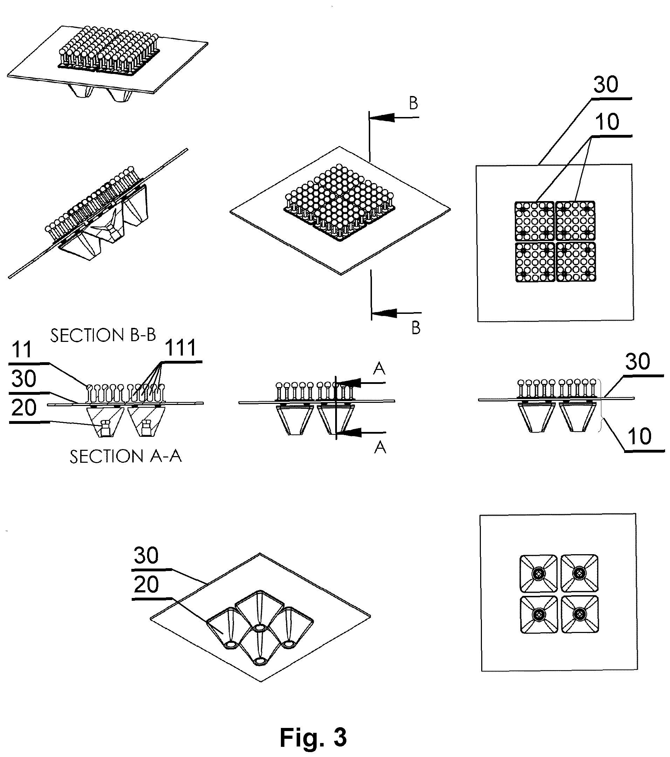

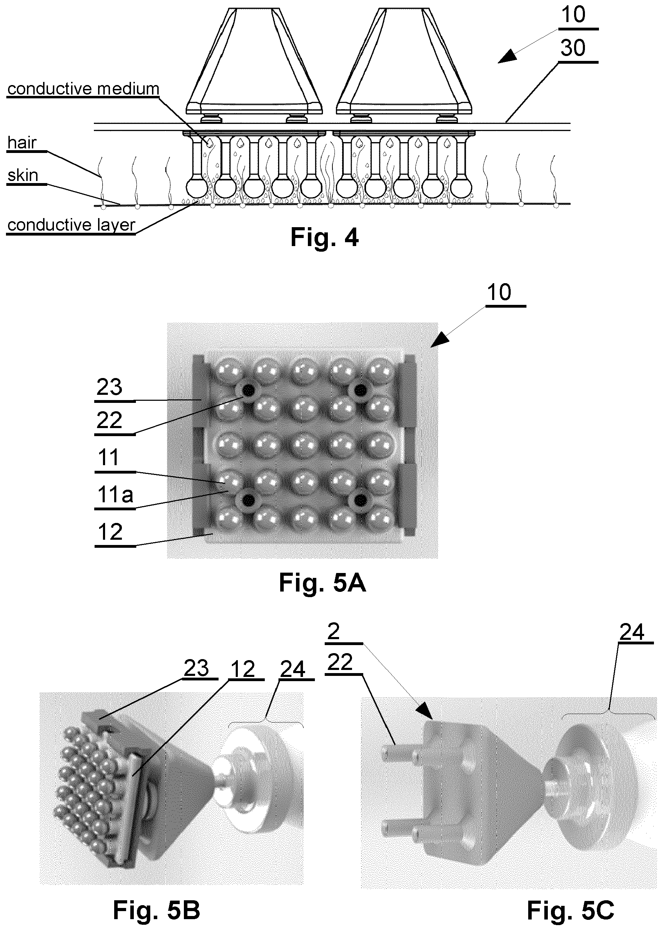

The surface electrode may comprise a plurality electrode units 10, e.g. two electrode units 10, three electrode units 10, four electrode units 10, or more than four electrode units 10, each unit 10 comprising a plurality of component electrodes 11, as shown in Fig. 3 and 4.

The component electrodes 11 of each electrode unit 10 protrude from the substrate 12 in the same direction, thereby forming a brush-like structure. Each component electrode 11 comprises a proximal end 11b connected with the substrate 12, a distal end 11a protruding outside the substrate 12, and a medium portion 11c between the distal end 11a and the proximal end 11b of the component electrode 11. The distal ends 11a together determine a working area of the surface electrode to be applied on the skin, at the desired treatment zone.

The layout of the component electrodes 11, within a single electrode unit 10, may take various forms, for example, a form of one or more than one row of component electrodes 11 further arranged in series or in parallel, as shown in Fig 1, in which the component electrodes 11 together form a square layout of sides: 5x5 component electrodes 11.

The distal ends 11a of the component electrodes 11may be of various shapes, preferably selected from the group consisting of sphere, hemisphere, roll, cylinder, stick-like, pin-like, spike-like, or pyramidal shape. The proximal ends 11b of the component electrodes 11 may be of various shapes, preferably selected from the group consisting of roll, cylinder or pyramidal shape. The medium portions 11c of the electrode components 11 may be in a shape of a solid of revolution, e.g. in a cylindrical shape. Preferably, within a single component electrode 11, the thickness (T) of its distal end 11a is greater than the thickness (t) of its medium portion 11c, as schematically shown in Fig 1D. The thickness may be expressed, for example, by size or radius of the distal end 11a, and the medium portion 11c, respectively. Thus, for example, for the circular or cylindrical shape of the distal end 11a, the radius of the distal end 11a of single component electrode 11 may be greater than the radius of its medium portion 11c, e.g. the radius of distal end 11a may be 1.5 mm and radius of medium portion 11c may be 0.5 mm. Greater thickness (T) of distal ends 11a leads to larger working area of the surface electrode, while smaller thickness (t) of medium portions 11c provides a respective free space volume 111 formed between the component electrodes 11 for hair. The free space volume 111 is thus restricted by the substrate 12 form one side and the distal ends 11a of the component electrodes 11 from another side. For example, the distal end 11a may have length of 3 mm and the medium portion 11c may have length of 6 mm. Alternatively, the length of medium portion may be increased to better suit users with longer and/or thicker hair. The length of distal end 11a may vary depending on its shape. Additionally, the radius of distal ends may be related to density of component electrodes 11 in electrode unit, i.e. the radius of distal end may be adjusted to provide specific spacing between two adjacent component electrodes 11, e.g. the spacing of 1 mm. Different spacing values are possible, however lower values may prevent the hair to move into free volume space 111, while higher values may lead to decrease of total contact area due to reduced number of component electrodes 11 per area unit. The component electrodes 11 may be made of various suitable materials, preferably electroconductive and biocompatible with the user's body. For example, the component electrodes 11 may be made of stainless steel, for instance, 316L stainless steel which provides good resistance to degradation processes and relatively low impedance. Another suitable example of the material for the component electrodes 11 is gold or silver coated with silver chloride. Alternatively, electroconductive polymers may be used.

Each electrode unit 10 is connectable, via connectors (not shown in the drawing for clarity) such as cables, to an output of a stimulation unit constituting a current and/or voltage supply so as to provide the electrical connection with each component electrode 11. This can be realized with various suitable means, such as used in the known surface electrode structures. Nonetheless, according to the present invention, each electrode unit 10 may be provided with an independent connector, thus, providing an independent electro-stimulation mode of each electrode unit 10. For example, during the performed electrostimulation, only selected electrode units 10 may be turned on, or provided with a different stimulation mode than the other electrode units 10, thereby simplifying adaptation of the working area of the surface electrode and adjusting the stimulation current-voltage of each electrode unit 10 with respect to the special user's needs.

Each electrode unit 10 further comprises a system 2 for supplying a conductive medium to the component electrodes 11 for forming a conductive layer between the distal ends 11a of the component electrodes 11 and skin, as shown schematically in Fig. 4. The system 2 for supplying a conductive medium of one electrode unit 10 may work independently from those of the other electrode units 10, or the systems for supplying a conductive medium of all the electrode units 10 of the surface electrode may work in co-operation, depending on the electrostimulation needs. The system 2 for supplying a conductive medium of each electrode unit 10 comprises a manifold 20, arranged on the back side 12b of the substrate, i.e. on the substrate side 12b that is opposite to the front side 12a with the protruding component electrodes 11. The manifold 20 is arranged opposite to the working area of the surface electrode so as to not disturb the operational condition of the surface electrode. The manifold 20 comprises ducts 21, each ended with the outlet tube 22 embedded through the substrate 12 material, across the substrate 12. Each outlet tube 22 delivers the conductive medium from the manifold 20, to the working area of the surface electrode, thus forming the conductive layer of the conductive medium, as shown in Fig. 4.

The conductive medium, preferably in a liquid state, may be fed to the manifold 20 from the conductive medium reservoir 24. The feeding may be accomplished by the use of any suitable means, such as a pump, pumping the conductive medium from the reservoir 24 to the manifold 20. In one embodiment of the working mode of the surface electrode, the conductive medium is delivered from the conductive medium reservoir to the component electrodes 11 only once per electrostimulation session, at the beginning of electrostimulation. In another embodiment of the working mode of the surface electrode, the conductive medium is delivered form the medium reservoir to component electrodes 11 through entire electrostimulation session, with predetermined dosage regime. The flow rate of the conductive medium, and thus the amount of the conductive medium dosed to the working area of the surface electrode, may be regulated by using various means, such as desired pump settings. The conductive medium may be preferably in a liquid state, e.g. the saline solution may be used, which has similar physical characteristics to body fluids, such as sweat. Furthermore, the system 2 for supplying a conductive medium may be configured for feeding the working area with less dense conductive medium, e.g. in form of aerosol, such as mixture of saline and air. Alternatively, the system may supply conductive medium e.g. in a gel state or similar.

Each electrode unit 10 may be provided with several outlet tubes 22, preferably evenly spaced across the substrate 12, as shown in Fig. 1A so as to deliver an equal amount of the conductive medium over the whole skin area that undergoes electrostimulation, thus, forming a continuous conductive layer. For example, one electrode unit 10 may comprise four outlet tubes 10, or more than four outlet tubes 10. In the case of very dense hair, the outlet tube 22 may be arranged between every two component electrodes 11 within the electrode unit 10. Preferably, the conductive medium is dosed dropwise, periodically or continuously, during the electrostimulation, depending on the needs. In some embodiments the conductive medium may be supplied under elevated pressure, to effectively and evenly spray the component electrodes 11, hair and the user's skin which is arranged under the surface electrode, during the electrostimulation.

The present invention is especially suitable for hairy skin regions. During the electrostimulation, the hair is disposed in the free space volume 111 between the component electrodes 11, i.e. over the distal ends 11a and between the medium portions 11c. The conductive medium is dosed dropwise from the outlet tubes 22 (not shown in Fig. 4 for clarity). The conductive medium forms the conductive layer between the distal ends 11a of the component electrodes 11 and the skin. The treatment may be carried out for the prolonged time, as the evaporating conductive medium is substituted with its new (fresh) portion, in the continuous or batch mode, depending on the need, thereby, keeping the proper thickness of the conductive layer during the whole treatment. Alternatively, during prolonged electrostimulation sessions, the conductive medium may be naturally substituted by the user's sweat, thus new portion of conductive medium may not be necessary. Impedance of the electrode-skin contact may be measured by stimulation unit to detect whether it is necessary to supply conductive medium during electrostimulation.

Furthermore, the surface electrode provides an increased contact area: electrode / conductive layer of conductive medium / skin as the developed construction enables the surface electrode to fit the body shape, e.g. head. This is achieved due to the presence of the electrode units 10 that are connected to each other via a deformable layer 30 of material. Alternatively, electrode units 10 may be attached to a substrate, wherein multiple substrates may be deformably connected to each other using mechanical joints.

In details, according to the present invention, the electrode units 10 are attached to the deformable layer 30 of material, such as fabric, foil, or flexible and/or ductile sheet of material. Further, the material constituting the deformable layer 30 may be a part of a cap or band, in particular of a headcap or a headband, serving to stabilize the surface electrode on the body part. The attachment of each electrode unit 10 to the deformable layer 30 is provided by sandwiching the deformable layer 30 by the substrate 12 of the electrode unit 10 from one side, and by the manifold 20 from another side of the deformable layer 30, as schematically shown in Fig. 3 and 4. The attachment may be accomplished for example by snap fasteners comprising a pair of interlocking parts, a male part and a female part. For example, the male parts 121 may be arranged on the substrate 12 in a form of hollow cylinders 121, arranged on the back side 12b and protruding out of the substrate 12 11, as schematically shown in Fig. 1D, 1E. The female parts of the snap fasteners may be arranged as the outlet tubes 22 of the manifold 20. Each outlet tube 22 and each hollow cylinder 121 may be complementary shaped to form the interlocking pair. Thus, in order to fasten the interlocking parts, the hollow cylinders 121 and the outlet tubes 22, the deformable layer 30 may comprise the apertures through which the hollow cylinders 121 are provided through the deformable layer, so as to join the substrate 12 and the manifold 20, with the deformable layer 30 sandwiched therebetween. Each hollow cylinder 121 comprise hollow body. Thus, upon fastening of the interlocking parts, the hollow cylinder 121 may form a jacket pipe of the outlet tube 22 enabling the conductive medium to flow through the outlet tube 22 undisturbed. The deformable layer 30 may have openings through which the hollow cylinders are transferred to be joined with the outlet tubes 22 of the system 2 for supplying a conductive medium.

Furthermore, the substrate 12 may be connected with the manifold 20 via other fastening means 23, e.g. fastening clips 23 shown in Fig. 5. In this configuration, the deformable layer 30 (not shown in Fig. 5 for clarity) may be also provided with the openings to transfer the connecting elements, e.g. clips of fastening means 23 through the material of the deformable layer.

The outlet tubes 22 of each electrode unit 10 are provided through the substrate 12 and, in the same manner, through the deformable layer 30. Thus, the conductive medium is supplied to the working area undisturbed. The electrode units 10, attached to the deformable layer 30, are spaced from each other so that, upon deformation of the deformable layer 30, the electrode units 10 may move relative to each other so as to fit the working area of the surface electrode to the user's body shape. The smaller the dimensions of each electrode unit, the greater fitting may be achieved. Thus, for the embodiment in which one electrode unit 10 comprises only one component electrode 11, and the surface electrode comprises a plurality of the electrode units 10, evenly distributed on the deformable layer 30, the most exact fitting may be achieved.

However, it is preferred to provide at least four, or more preferably at least nine component electrodes 11 in a single electrode unit 10. This provides better stability of the electrode unit 10 as each component electrode 11 provides one point of contact with the skin.

The component electrodes 11 may constitute an integral part of the substrate 12, with both the substrate 12 and the component electrodes 11 made of one piece of material, e.g. stainless steel. For example, the substrate 12 and the component electrodes 11 may be 3D printed as one integral part.

Alternatively, the substrate 12 and the component electrodes 11 may be made of individual parts, which are joined together. For example, the substrate 12 may be made of a rigid material, such as rigid plastic or metal plate, thus ensuring the rigidity of a single electrode unit 10 and at the same time deformability of the whole surface electrode, wherein the latter is ensured by the deformable layer 30. The application of rigid substrates 12 provides good stability of the component electrodes 11 within a single electrode unit 10.

Nonetheless, the substrate 12 may be made of flexible material, such as flexible plastic plates. The flexibility of the substrate 12 may provide a better fit of the electrode to the body shape.

## Claims

1. A surface electrode for application on a skin, the surface electrode comprising:
- electrode units (10) connectable to a voltage and/or current supply, each electrode unit (10) comprising:
- a substrate (12) having a front side (12a) and a back side (12b), and
- component electrodes (11) disposed on the front side (12a) of the substrate (12) and spaced apart from each other such that a free space volume (111) is formed between the component electrodes (11), each component electrode (11) having a proximal end (11b) connected with the substrate (12), a distal end (11a) configured to be applied on the skin, and a medium portion (11c) between the proximal end (11b) and the distal end (11a).

2. The surface electrode according to claim 1 further comprising a system (2) for supplying a conductive medium to the free space volume (111), the system comprising a manifold (20) arranged on the back side (12b) of the substrate (12), the manifold (20) comprising ducts (21) ended with outlet tubes (22) arranged in the substrate (12) from the back side (12b) to the front side (12a) for supplying the conductive medium to the component electrodes (11).

3. The surface electrode according to claim 1 or 2 wherein the electrode units (10) are arranged on a deformable layer (30) that is disposed between the manifold (20) and the substrate (12) facing the back side (12b) of the substrate.

4. The surface electrode according to claim any of the preceding claims wherein the deformable layer (30) has openings for disposing the outlet tubes (22) from one side of the deformable layer (30) to the other side.

5. The surface electrode according to any of the preceding claims wherein the manifold (20) is connected with the substrate (12) by fastening means (23, 121).

6. The surface electrode according to any of the preceding claims comprising at least two electrode units (10).

7. The surface electrode according to any of the preceding claims wherein one electrode unit (10) comprises at least four component electrodes (11).

8. The surface electrode according to any of the preceding claims wherein the component electrodes (11) comprise:
- the distal ends (11a) of a distal end thickness (T) having a shape selected from the group consisting of sphere, hemisphere, roll, cylinder, stick-like, pin-like, spike-like, and pyramidal shape,
- the medium portions (11c) of a medium portion thickness (t), and
- the proximal ends (11b) having a shape selected from the group consisting of roll, cylinder, stick-like, pin-like, spike-like, and pyramidal shape,
wherein, within one electrode component (11) the distal end thickness (T) is greater than the medium portion thickness (t).

9. The surface electrode according to any of the preceding claims wherein the component electrodes (11) are made of stainless steel or chloride plated with gold or with silver.

10. The surface electrode according to any of the preceding claims wherein the system for supplying a conductive medium is connected with a reservoir (24) for conductive medium.

11. The surface electrode according to claim 10 wherein the system for supplying a conductive medium comprises a pump for feeding the conductive medium from the reservoir (24) to the outlet tubes (22).

12. The surface electrode according to any of the claims from 3 to 11 wherein the deformable layer (30) is made of at least one material selected from the group consisting of cotton, polyamide, polyester and leather.

13. The surface electrode according to any of the preceding claims wherein the component electrodes (11) are an integral part of the substrate (12).

14. The surface electrode according to any of the claims from 1 to 12 wherein the component electrodes (11) are attached to the substrate (12).
